Europäisches Patentamt

European Patent Office

Office européen des brevets

(19)

(11) Publication number: **0 237 338 B1**

(12) **EUROPEAN PATENT SPECIFICATION**

(45) Date of publication of patent specification: **23.09.92**

(51) Int. Cl.⁵: **C12N  15/72**, C12N 1/20, C12P 21/02, //(C12N1/20, C12R1:19)

(21) Application number: **87302093.7**

(22) Date of filing: **11.03.87**

(54) Hybrid expression vectors, their construction and uses.

(30) Priority: **12.03.86 US 838772**

(43) Date of publication of application:
**16.09.87 Bulletin  87/38**

(45) Publication of the grant of the patent:
**23.09.92 Bulletin  92/39**

(84) Designated Contracting States:
**AT BE CH DE ES FR GB GR IT LI LU NL SE**

(56) References cited:
**EP-A- 0 067 540**
**EP-A- 0 108 387**
**EP-A- 0 152 830**
**EP-A- 0 211 299**

(73) Proprietor: **INTERNATIONAL MINERALS AND CHEMICAL CORPORATION**
**P.O. Box 207 1401 South Third Street**
**Terre Haute Indiana 47808(US)**

(72) Inventor: **Maccecchini, Maria-Luisa**
**c/o Bachem AG Hauptstrasse 144**
**CH-4416 Bubendorf(CH)**

(74) Representative: **Holmes, Michael John et al**
**Frank B. Dehn & Co. European Patent Attorneys Imperial House 15-19 Kingsway**
**London, WC2B 6UZ,(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to hybrid expression vectors, their construction and uses.

Expression vectors (e.g., plasmids) for the expression of inserted DNA in microorganisms (e.g., E. coli) are known in the art, and are commercially available from several sources. Such vectors typically include a promotor and operator region including transcription and translation control signals, downstream from which region is located one or more restriction endonuclease sites which are preferably unique to the vector. The restriction site or sites are positioned downstream of the promotor and operator region to provide a location for insertion into the vector of a DNA segment of interest for expression of the DNA under the control of the promotor and operator region.

In attempting to insert DNA segments into expression vectors for expression of the DNA, certain difficulties may be encountered. For example, if the promotor or other crucial region of the vector (e.g., origin of replication, phenotypic marker, etc.) is located between two unique but different restriction endonuclease sites of the vector, and a DNA segment of interest is bounded by the same two restriction sites, cutting the vector at the two unique restriction sites for insertion of the DNA segment removes the promotor region (or other critical region) from the vector and destroys the usefulness of the vector as an expression vector. Loss of the promotor (or other critical region) can be avoided with such a vector by modifying the DNA segment of interest and inserting the segment into a single unique restriction site of the vector located downstream of the promoter region. One method of modifying the DNA segment of interest involves altering the DNA segment so that the segment is bounded by identical restriction sites which match a unique restriction site downstream of the promoter region of the vector. However, difficulties are encountered when inserting a DNA segment bounded by identical restriction sites into a unique matching restriction site of a vector. One problem is that the DNA segment of interest can be inserted into the vector in two orientations, only one orientation of which can result in the desired expression product coded for by the DNA segment. Another problem is that after opening the vector at the unique restriction site, the vector can recircularize before insertion of the DNA segment of interest takes place.

In accordance with the present invention, there is provided the plasmid designated pML3 (as present in E. coli strain JM103 (pML3) deposited with the American Type Culture Collection, Rockville, Maryland as ATCC. No. 53377). Such a hybrid plasmid comprises a hybrid trp/lac (tac) promoter and operation region including transcription and translation control signals, proximally downstream of which region is located a unique EcoRI restriction site, downstream of which EcoRI site is located a unique BamHI restriction site. The plasmid further comprises a selectable phenotypic marker and a functional replicon for E. coli, the promoter and operator region being capable of expressing DNA inserted between the EcoRI site and the BamHI site. The invention further relates to microorganisms transformed by such a hybrid plasmid, a method for expressing DNA utilizing such a hybrid plasmid, and a method for constructing such a hybrid plasmid.

## BRIEF DESCRIPTION OF THE DRAWINGS

FIG. 1 is a schematic diagram of plasmid starting material which can be used to construct a vector according to the present invention.

FIG. 2 is a schematic diagram showing deletion of an EcoRI site from the plasmid shown in FIG. 1.

FIG. 3 is a schematic diagram illustrating restriction site conversion of the plasmid shown in FIG. 1 after removal of the EcoRI site.

## DESCRIPTION OF THE PREFERRED EMBODIMENTS

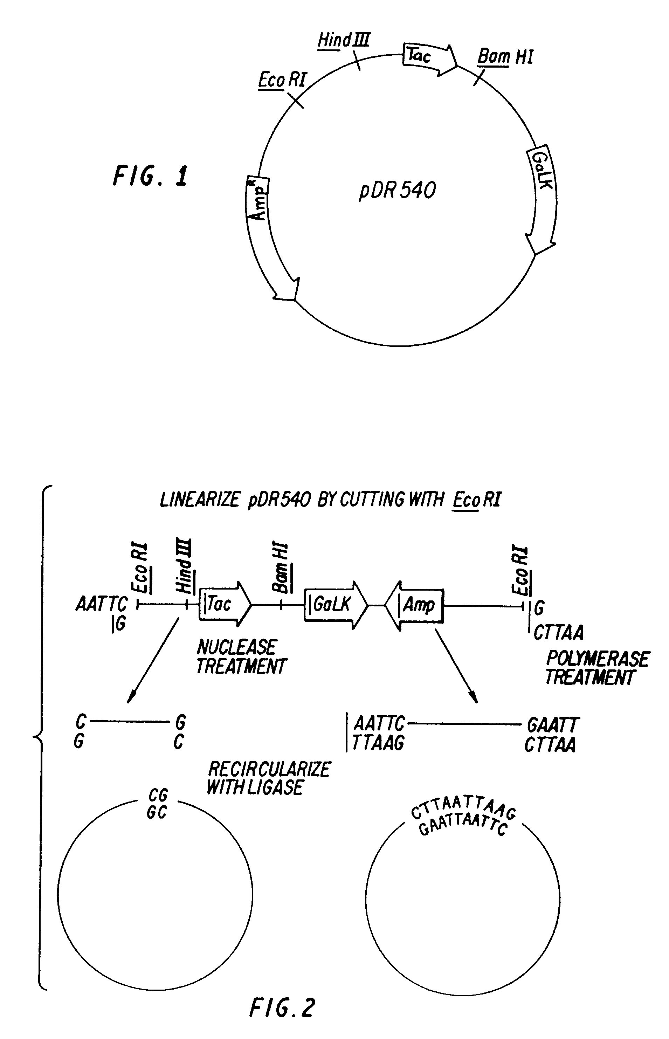

A suitable starting material for construction of a hybrid expression plasmid according to the present invention is plasmid pDR540, which is commercially available from PL Biochemicals, Inc., Milwaukee, Wisconsin. See Fig. 1. While the invention will be further described with reference to plasmid pDR540 as the starting material, it is to be understood that any suitable plasmid possessing similar characteristics as pDR540 can be used as a starting material.

Plasmid pDR540 is a multicopy expression vector derived from plasmid pK0-1 (McKenney, K. et al, Gene Amplification and Analysis, Vol. II (Chirikjian, J.G. and Papas, T., ed.) Elsevier, North Holland, p. 383 (1981)). Plasmid pDR540 includes a trp/lac (tac) promotor and operator region proximally downstream from which is located a BamHI site unique to the plasmid (deBoer, H. et al., Proc. Natl. Acad. Sci. USA, 80:21 (1983) and Russel, D.R. and Bennett, G.N., Gene, 20:321 (1982). The unique BamHI site of pDR540 is in position for expression of DNA inserted therein under the control of the hybrid tac promotor and operator region. Plasmid pDR540 includes an expressible nucleotide sequence coding for GalK downstream of the unique BamHI site, the GalK sequence being under the control of the tac promoter.

Plasmid pDR540 has a unique EcoRI site upstream of the tac promoter, and a selectable phenotypic maker in the form of an expressible nucleotide sequence coding for amp$^r$, the amp$^r$ sequence being upstream of the EcoRI site and the tac promoter. The amp$^r$ sequence has an opposite direction of transcription relative to the GalK sequence. Plasmid pDR540 also includes a functional replicon for E. coli, permitting replication of the plasmid in E. coli independent of chromosomal replication.

Plasmid pDR540 is not particularly well suited for the insertion and expression of DNA segments bounded by an EcoRI site and a BamHI site (such as a growth hormone-releasing factor (GRF) gene bounded by a 5′ EcoRI site and a 3′ BamHI site). Endonuclease digestion of pDR540 with EcoRI and BamHI cuts the hybrid tac promoter region from the plasmid and destroys the plasmid's usefulness as an expression vector.

A hybrid plasmid according to the present invention can be constructed using plasmid pDR540 by first removing the unique EcoRI restriction site located upstream of the hybrid tac promoter. See Fig. 2.

According to one embodiment, the EcoRI site is removed from pDR540 by linearizing the plasmid with restriction endonuclease EcoRI, filling in the resultant sticky ends (Klenow fragment) to form a flush (blunt) ended molecule, and recircularizing the plasmid by bluntend ligation to form pDR540 (-EcoRI).

In a second embodiment, the unique EcoRI site of pDR540 is removed by linearizing the plasmid with EcoRI, chewing away the sticky ends with an exonuclease to produce a flush-ended molecule, and recircularizing the plasmid by blunt-end ligation to form pDR540 (-EcoRI).

Following the removal of the EcoRI site from pDR540, the BamHI site downstream of the hybrid tac promoter is modified to form an EcoRI-BamHI site. This can be accomplished by inserting a suitable DNA segment into the BamHI site of pDR540 (-EcoRI). A suitable DNA segment for insertion into the BamHI site of pDR540 (-EcoRI) is bounded on its 5′ end by a cut Sau3A restriction site, and bounded on its 3′ end by a cut BamHI restriction site. The segment also includes an intact and unique EcoRI restriction site which preferably is adjacent to or in close proximity to the 5′ cut Sau3A site.

The 5′ end of the Sau3A-BamHI segment has a sticky end which is complementary to the 3′ end of BamHI linearized pDR540 (-EcoRI). However, a Sau3A restriction site cannot be recognized and cut by BamHI restriction endonuclease. Thus, by inserting the Sau3A-BamHI segment into the BamHI site of pDR540 (-EcoRI), a Sau3A site is created at the 3′ portion of the BamHI site of pDR540 (-EcoRI) which is not recognized by BamHI. However, the BamHI site at the 5′ portion of the BamHI site of pDR540 (-EcoRI) remains intact. Insertion of the Sau3A-BamHI segment into the BamHI site of pDR540 (-EcoRI) provides the plasmid with a unique EcoRI restriction site proximally downstream from the tac promoter, and with a unique BamHI site located downstream of the inserted EcoRI site.

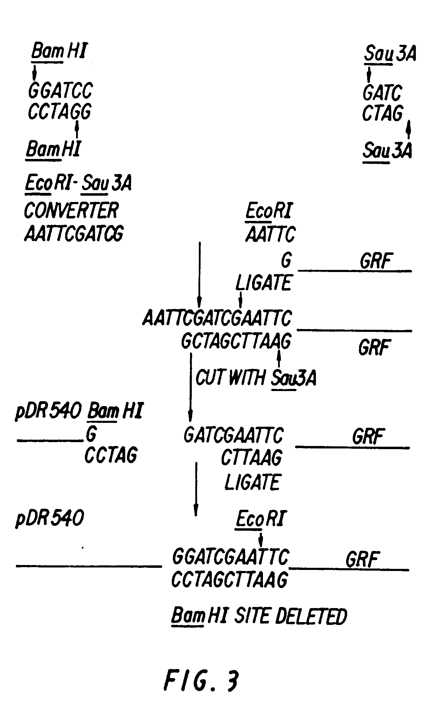

A suitable Sau3A-BamHI segment can be constructed using a GRF gene (supra) bounded 5′ by an EcoRI site and bounded 3′ by a BamHI. See Fig. 3. A pair of EcoRI-Sau3A convertors having the sequence AATTCGATCG are ligated to the 5′ end of an EcoRI-cut GRF gene. The convertor DNA then is cut with Sau3A and the 3′ end of the GRF segment is cut with BamHI. The Sau3A-BamHI GRF segment is then inserted into the BamHI site of pDR540 (-EcoRI) to form plasmid pML3. Plasmid pML3 has a unique inserted EcoRI site 36 base pairs downstream of the tac promoter, and a unique BamHI site downstream of the inserted EcoRI site. Plasmid pML3 has been used to transform E. coli strain JM103, a lacI$^Q$ host (Δ (lacipro), thi, strA, supE, endA, sbcB, hsdR, F′[traD36, proAB, laci$^Q$, ZΔM15]), the transformed strain being designated Bacterium JM103 (pML3) and deposited with American Type Culture Collection, Rockville, Maryland as ATCC No. 53377.

E. coli cells transformed by pML 3 can be cultivated to express the GRF gene. Furthermore, any DNA segment which is flanked by EcoRI and BamHI can be inserted into EcoRI/BamHI-cut pML3 according to the present invention. Such insertions are unidirectional, unambiguous and take place without recircularization of the plasmid. A DNA segment so inserted is located proximally downstream of the hybrid tac promoter for optimal expression of the inserted DNA by transformed E. coli microorganisms which are cultivated under protein-producing conditions (e.g., induction by IPTG or X-gal).

The invention is further illustrated by the following examples which are not intended to be limiting.

Example I

Elimination of the EcoRI site of Plasmid pDR540 (Fig. 2)

Plasmid pDR540 (supra) was linearized with restriction endonuclease EcoRI (10,000 units/ml; Bethesda Research Laboratories) according to manufacturer's instructions to produce linearized DNA molecules have recessed (sticky) ends. The EcoRI site of EcoRI linearized pDR540 was removed by each of methods (a), (b) and (c) below.

(a) Polymerization of Recessed Ends of EcoRI lin-

earized pDR540

1 $\mu$g of EcoRI linearized pDR540 DNA with recessed ends was mixed with 5 $\mu$l of the 10 x nick translation buffer (0.5 M Tris-HCl pH 7.2, 0.1 M MgSO4, 1 mM DTT (Dithiothreitol, ultra pure, International Biotechnologies Inc.), 0.5 mg/ml BSA (bovine serum albumin, Sigma), 2 nM of each of dATP and dTTP (the deoxynucleotides necessary to fill in the recessed 3' ends resulting from EcoRI digestion), 1 unit/$\mu$g DNA Klenow fragment (E. coli DNA polymerase I Klenow, 4500 units/ml, Pharmacia), and sterile water, to a volume of 50 $\mu$l 0.5M EDTA. The flush-ended DNA was extracted with phenol/chloroform, and precipitated by making mix 2M in acetate and adding 2 volumes of isopropanol, incubating the mixture at room temperature for 10 min., spinning the mixture in microfuge for 10 min., air-drying the precipitated pellet, and resuspending the pelleted DNA in water.

(b) Mung Bean Nuclease Digestion of EcoRI Linearized pDR540

1 $\mu$g of EcoRI linearized pDR540 DNA with recessed ends was mixed with 25 mM NH$_4$OAc pH 5.0, and 2 units of mung bean nuclease (Mung bean nuclease 100,000 units/ml P-L Biochemicals) for 30 min. at 30°C. The reaction was stopped by adding 110 mM K phosphate pH 70, 100 mM NaCl, and heating at 72°C for 15 min., followed by chilling to 0°C. The flush-ended DNA was then ethanol precipitated, microfuged to form a pellet, which was air-dried, and the pellet was resuspended in water.

(c) SI Nuclease Digestion of EcoRI Linearized pDR540

1 $\mu$g of EcoRI linearized pDR540 was incubated in 0.03 M Na acetate, pH 4.6, 0.3 M NaCl, 4.5 mM ZnCl, and 5 units S1 nuclease (S1 nuclease, 250,000 units/ml, BRL) at 22°C for 30 min. followed by 15 min. at 10°C. The digestion was stopped by addition of Tris base to 0.1 M, EDTA to 25 mM. The flush-ended DNA was then ehtanol precipitated, microfuged to form a pellet which was air-dried, and the pellet was resuspended in water.

(d) Blunt End Ligation

1$\mu$g of the resuspended flush-ended DNA in water from procedures (a), (b) and (c) above were individually mixed with 10x ligase buffer (0.3 M Tris-HCl, pH 8.0, 40 mM MgCl$_2$, 10 mM EDTA, 0.1 M DTT, 0.5 mg/ml BSA), 0.5 mM ATP, 5 units DNA ligase/$\mu$g DNA (E. coli T4 DNA ligase, 3000/ml units; P-L Biochemicals), and sterile water to obtain final DNA concentrations of 0.5-1 $\mu$g DNA/20 $\mu$l mix. The ligation mixtures were incubated overnight at 14°C. The mixtures were adjusted to 0.25 M in Na acetate, and the recircularized DNA was precipitated from each mixture with 2.5 volumes EtOH. The precipitated pellets of recircularized DNA from procedures a, b and c were then individually resuspended in water.

(e) Transformation

DNA suspensions from procedures (a), (b) and (c) above which had been recircularized according to procedure (d) above were individually digested with EcoRI which site was to be deleted from the DNA, to eliminate any plasmids still containing the site. The digestion mixes were individually isopropanol precipitated by making them 20 mM in EDTA, 2 M in ammonium acetate, adding 2 volumes isopropanol, and incubating at room temperature for 10 min. The mixtures were spun at room temperature for 5 min., air dried, and resuspended in small volumes of TE (1 $\mu$g DNA/10 $\mu$l TE:10 mM Tris-HCl pH 7.9, 1 mM EDTA). E. coli cells were transformed with the samples, colonies were harvested, and plasmid DNA was screened for deletion of the EcoRI restriction endonuclease site by restriction mapping.

Example II

Confirmation of deletion of the EcoRI site from pDR540

Flush-ended DNA from Example I(a) which had been blunt-end ligated as in Example I(d) was sequenced according to the procedure of Maxam and Gilbert, Proc. Natl. Acad. Sci. USA, 74:560 (1977) and the sequence was confirmed by the dideoxy sequencing procedure of Sanger et al., Proc. Acad. Sci. USA, 74: 5403 (1977). The data confirmed the deletion of the EcoRI site by polymerase I Klenow fragment (Example I(a)). However, instead of the expected sequence with the doubling of the EcoRI sticky ends, the polymerase had degraded one nucleotide from one end to give the sequence -GAATAATTC- instead of the expected -GAATTAATTC-. This deletion does not affect the functioning of the plasmid since it is not in a reading frame and it is not in an otherwise important part of the plasmid. The plasmid is designated pDR540 (-EcoRI).

Example III

Conversion of BamHI site of pDR540 (-EcoRI) to an EcoRI-BamHI site (Fig. 3)

Plasmid pUC9 Vieira et al., Gene, 19:259 (1982) and commercially available from Pharmacia P-L Biochemicals, containing a nucleotide sequence coding for GRF (supra) bounded by a 5′ EcoRI site and a 3′ BamHI site was cut out of pUC9 by digestion with EcoRI and BamHI endonucleases under the following conditions: 1 μg of pUC9 DNA in high salt buffer (10 mM Tris-HCl (pH 8.0), 100 mN NaCl, 10 mM MgCl₂, 1 mM β-mercaptoethanol, 100 μg BSA/ml) was mixed with 1 unit EcoRI and 1 unit BamHI. The mixture was incubated one hour at 37°C, and the cut EcoRI-BamHI GRF segment was isolated by gel electrophoresis and electroelution.

Dephosphorylated EcoRI-Sau3A convertors having the sequence AATTCGATCG were phosphorylated as follows: 1 μg dephosphorylated convertor DNA was mixed with 10 μl 10 x kinase buffer I (0.5 M Tris-Cl (pH 7.6), 0.1 M MgCl₂, 50 mM dithiothreitol, 1 mM spermidine, 1 mM EDTA), 50 pmoles (200 μ Ci) [γ-³²P] ATP (sp. act. = 3,000 in/mmole), 10 units T4 polynucleotide kinase and H₂O to 50 μl. The mixture was incubated at 37°C for 30 minutes. To the incubated mixture was added 2 μl of 0.5 M EDTA, convertor DNA was extracted once from the mixture with phenol/chloroform, and the convertor DNA was precipitated with ethanol. The ethanol precipitated DNA was redissolved in 50 μl of TE (pH 7.9), and phosphorylated convertor DNA was separated from the redissolveed DNA by centrifugation through small columns of Sephadex G-50.

The EcoRI-BamHI cut GRF DNA was mixed with phosphorylated convertor DNA to ligate the convertors to the GRF DNA under the conditions set forth in Example I(d) except that 1 unit DNA ligase/μg DNA was present in the reaction mixture. The GRF gene with convertor attached was digested with Sau3A and BamHI enzymes, under the same conditions set forth above with respect to the EcoRI/BamHI double digest of pUC9, but with Sau3A enzyme substituted for EcoRI enzyme, to give a GRF gene segment bounded by a 5′ cut Sau3A site and a 3′ cut BamHI site, with an intact EcoRI site adjacent the Sau3A site (termed Sau3A-BamHI GRF segment).

Plasmid pDR540 (-EcoRI) (Example II, supra) was linearized with 1 unit BamHI enzyme/μg DNA (per manufacturer's instructions), and the linearized DNA was dephosphorylated as follows:

BamHI linearized plasma was extracted once with phenol/chloroform and precipitated with ethanol. The precipitated DNA was dissolved in a minimum volume of 10 mM Tris-Cl (pH 8.0), to which was then added 5 μl 10x CIP (calf intestional alkaline phosphatase) buffer (0.5 M Tris-Cl (pH 9.0), 10 mM MgCl₂, 1 mM ZmCl₂, 10 mM spermidine), 0.01 unit CIP and H₂O to 48 μl. The mixture was incubated at 37°C for 30 minutes, a second aliquot of 0.01 unit CIP was added to the mixture, and the incubation was continued an additional 30 minutes. To the incubated mixture was then added 40 μl H₂O, 10 μl 10x STE (100 mM Tris-Cl (pH 8.0), 1 M NaCl, 10 mM EDTA) and 5 μl 10% SDS. This mixture was then incubated at 68°C for 15 minutes. Dephosphorylated DNA was extracted twice with phenol/chloroform and twice with chloroform, then precipitated with ethanol. 1 μg of BamHI cut dephosphorylated pDR540 (-EcoRI) was mixed with 1 μg Sau3A-BamHI GRF segment and 2 units DNA ligase to ligate the segments and form plasmid pML3. The ligation conditions were otherwise as set forth above for ligation of convertor DNA to EcoRI cut GRF DNA. Plasmid pML3 has a unique inserted EcoRI site 35 base pairs downstream of the tac promoter and a unique BamHI site downstream of the inserted EcoRI site. E. coli strain JM103 has been transformed by plasmid pML3 and deposited with American Type Culture Collection, Rockville, Maryland as ATCC No. 53377.

## Claims
### Claims for the following Contracting States : BE, CH, DE, FR, GB, GR, IT, LI, LU, NL, SE

1. The plasmid designated pML3 (as present in E. coli strain JM103 (pML3) deposited with the American Type Culture Collection, Rockville, Maryland as ATCC. No. 53377).

2. A microorganism transformed by the plasmid as claimed in claim 1.

3. A method for expressing a DNA sequence comprising inserting said DNA sequence between the EcoRI site and the BamHI site of a plasmid as claimed in claim 1, transforming a microorganism with said plasmid and cultivating the microorganism so as to express said DNA sequence.

4. A method for expressing a GRF gene comprising cultivating a microorganism transformed with the plasmid of claim 1 so as to express the GRF gene therein present

5. A method for constructing a hybrid plasmid as claimed in claim 1 characterised in that a plasmid comprising a hybrid trp/lac (tac) promoter and operator region including transcription and translation control signals, proximally downstream of which region is located a unique BamHI restriction site, the plasmid further comprising a selectable phenotypic marker and a functional replicon for E.coli, is treated to insert into said BamHI site a DNA sequence bounded by a 5' cut Sau 3A restriction site and a 3' cut BamHI restriction site and including a unique EcoRI restriction site.

6. A method as claimed in claim 5 comprising:
(a) linearizing a plasmid with restriction endonuclease EcoRI to produce sticky ends, the plasmid having a unique EcoRI restriction site located upstream of a hybrid trp/lac (tac) promoter and operator region including transcription and translation control signals, a unique BamHI restriction site proximally downstream of said region, this region being capable of expressing DNA inserted into the BamHI site, the plasmid further comprising a selectable phenotypic marker and a functional replicon for E. coli;
(b) blunting the sticky ends to provide a linearized plasmid with flush ends;
(c) blunt-end ligating the linearized plasmid of step (b) to provide a circularized plasmid without an EcoRI site; and
(d) inserting into the BamHI site of the circularized plasmid of step (c) a DNA segment bounded by a 5' cut Sau3A restriction site and a 3' cut BamHI restriction site and including a unique EcoRI restriction site.

7. A method as claimed in claim 5 or claim 6, wherein the starting plasmid and the final hybrid plasmid further comprise an expressible nucleotide sequence coding for GalK downstream of said BamHI site, the GalK sequence being under the control of said promoter and operator region.

8. A method as claimed in claim 6, wherein the selectable phenotypic marker of the starting plasmid and the final hybrid plasmid is an expressible nucleotide sequence coding for amp$^r$, said amp$^r$ sequence being upstream of said promoter and having an opposite direction of transcription from the GalK sequence.

9. A method as claimed in any one of claims 5 to 8, wherein the EcoRI site of the final hybrid plasmid is about 36 base pairs downstream of said promoter.

10. A method as claimed in claim 9 wherein the final hybrid plasmid is pML3 (present in E. coli strain JM103 (pML3), deposited with the American Type Culture Collection, Rockville, Maryland as ATCC No. 53377).

**Claims for the following Contracting States : AT, ES**

1. A method of constructing a hybrid plasmid for expressing a DNA sequence characterised in that a plasmid comprising a hybrid trp/lac promoter and operator region including transcription and translation control signals, proximally downstream of which region is located a unique BamHI restriction site, the plasmid further comprising a selectable phenotypic marker and a functional replicon for E.coli, is treated to insert into said BamHI site a DNA sequence bounded by a 5' cut Sau3A restriction site and a 3' cut BamHI restriction site and including a unique EcoRI site
so as to provide a hybrid plasmid designated pML3 (as present in E.coli strain JM103 (pML3), deposited with the American Type Culture Collection, Rockville, Maryland as ATCC No. 53377), and having a unique EcoRI restriction site proximally downstream of said promoter and a unique BamHI site downstream of said EcoRI site, said promoter and operator region being capable of expressing DNA inserted between the EcoRI and BamHI sites.

2. A method as claimed in claim 1 comprising:
(a) linearizing a plasmid with restriction endonuclease EcoRI to produce sticky ends, the plasmid having a unique EcoRI restriction site located upstream of a hybrid trp/lac (tac) promoter and operator region including transcription and translation control signals, a unique BamHI restriction site proximally downstream of said region, this region being capable of expressing DNA inserted into the BamHI site, the plasmid further comprising a selectable phenotypic marker and a functional replicon for E. coli;
(b) blunting the sticky ends to provide a linearized plasmid with flush ends;
(c) blunt-end ligating the linearized plasmid of step (b) to provide a circularized plasmid without an EcoRI site; and
(d) inserting into the BamHI site of the circularized plasmid of step (c) a DNA segment bounded by a 5' cut Sau3A restriction site and a 3' cut BamHI restriction site and including a unique EcoRI restriction site.

3. A method as claimed in claim 1 or claim 2, wherein the starting plasmid and the final hybrid plasmid further comprise an expressible nucleotide sequence coding for GalK downstream of said BamHI site, the GalK sequence being under the control of said promoter and operator region.

4. A method as claimed in claim 3, wherein the selectable phenotypic marker of the starting plasmid and the final hybrid plasmid is an expressible nucleotide sequence coding for amp$^r$, said amp$^r$ sequence being upstream of said promoter and having an opposite direction of transcription from the GalK sequence.

5. A method as claimed in any one of claims 1 to 4 wherein the EcoRI site of the final hybrid plasmid is about 36 base pairs downstream of said promoter.

6. A method as claimed in any one of claims 1 to 5 wherein the starting plasmid and the final hybrid plasmid are multicopy plasmids.

7. A microorganism transformed by the plasmid pML3 and designated E. coli strain JM103 (pML3), deposited with the American Type Culture Collection, Rockville, Maryland as ATCC No. 53377.

8. A method for expressing a DNA sequence comprising inserting said DNA sequence between the EcoRI site and the BamHI site of a plasmid obtainable by a method as claimed in any one of claims 1 to 6, transforming a microorganism with said plasmid and cultivating the microorganism so as to express said DNA sequence.

9. A method for expressing a GRF gene comprising cultivating a microorganism transformed with the plasmid designated pML3 (as deposited in E. coli strain JM103 (pML3) with the American Type Culture Collection, Rockville, Maryland as ATCC No. 53377) so as to express the GRF gene therein present.

**Patentansprüche**
**Patentansprüche für folgende Vertragsstaaten : BE, CH, DE, FR, GB, GR, IT, LI, LU, NL, SE**

1. Plasmid mit der Bezeichnung pML3 (vorliegend in dem E. Coli-Stamm JM103 (PML3), hinterlegt bei der American Type Culture Collection, Rockville, Maryland unter der Bezeichnung ATCC Hr. 53377).

2. Mikrorganismus, transformiert mit dem Plasmid gemäß Anspruch 1.

3. Verfahren zur Expression einer DNA-Sequenz, wobei man die DNA-Sequenz zwischen der EcoRI-Schnittstelle und der BamHI-Schnittstelle des Plasmids gemäß Anspruch 1 insertiert, einen Mikroorganismus mit dem Plasmid transformiert und den Mikroorganismus kultiviert, so daß die DNA-Sequenz exprimiert wird.

4. Verfahren zur Expression eines GRF-Gens, wobei man einen Mikroorganismus kultiviert, der mit dem Plasmid gemäß Anspruch 1 transformiert ist, so daß das darin vorliegende GRE-Gen exprimiert wird.

5. Verfahren zur Konstruktion eines Hybridplasmids gemäß Anspruch 1, dadurch gekennzeichnet, daß man ein Plasmid, welches eine Hybrid-trp/lac (tac)-Promotor- und Operatorregion einschließlich Transcriptions- und Translationskontrollsignalen umfaßt, wobei in proximaler Position stromabwärts von dieser Region eine einzige BamHI-Schnittstelle angeordnet ist, und das Plasmid außerdem einen selektierbaren, phänotypischen Marker und ein funktionales Replikon für E. coli umfaßt, behandelt, um in die BamHI-Schnittstelle eine DNA-Sequenz zu insertieren, weiche von einer 5'-geschnittenen Sau 3A-Schnittstelle und einer 3'-geschnittenen BamHI-Schnittstelle begrenzt ist und eine einzige EcoRI-Schnittstelle enthält.

6. Verfahren nach Anspruch 5, wobei man:
   (a) ein Plasmid mit der Restriktionsendonuklease EcoRI linearisiert, um klebrige Enden zu produzieren, wobei das Plasmid eine einzige EcoRI-Schnittstelle aufweist, die stromaufwärts von der Hybrid-trp/lac (tac)-Promotor- und Operatorregion liegt und Transcriptions- und Translations-Kontrollsignale enthält, eine einzige BamHI-Schnittstelle in proximaler Position stromabwärts von dieser Region aufweist, wobei diese Region in die BamHI-Schnittstelle insertierte DNA exprimieren kann, und wobei das Plasmid außerdem einen selektierbaren, phänotypischen Marker und ein funktionales Replikon für E. coli umfaßt;
   (b) die klebrigen Enden glättet, um ein linearisiertes Plasmid mit bündigen Enden zu erzeugen;
   (c) das linearisierte Plasmid gemäß Schritt (b) an den glatten Enden ligiert, um ein zirkularisiertes Plasmid ohne EcoRI-Schnittstelle zu erzeugen; und
   (d) in die BamHI-Schnittstelle des zirkularisierten Plasmids gemäß Schritt (c) ein DNA-Segment insertiert, welches durch eine 5'-geschnittene Sau3A-Schnittstelle und eine 3'-geschnittene BamHI-Schnittstelle begrenzt ist und eine einzige EcoRI-Schnittstelle aufweist.

7. Verfahren nach Anspruch 5 oder Anspruch 6, wobei das Ausgangsplasmid und das produzierte Hybridplasmid außerdem eine exprimierbare, für GalK kodierende Nukleotidsequenz stromabwärts von der BamHI-Schnittstelle aufweisen, wobei die GalK-Sequenz unter der Kontrolle der Promotor- und Operator-Region steht.

8. Verfahren nach Anspruch 6, worin der selektierbare, phänotypische Marker in dem Ausgangsplasmid und dem produzierten Hybridplasmid eine für amp$^r$ kodierende Nukleotidsequenz ist, wobei die amp$^r$-Sequenz stromaufwärts von dem Promotor liegt und eine im Vergleich zur GalK-Sequenz umgekehrte Transcriptionsrichtung aufweist.

9. Verfahren nach einem der Ansprüche 5 bis 8, worin die EcoRI-Schnittstelle des produzierten Hybridplasmids etwa 36 Basenpaare stromabwärts von dem Promotor liegt.

10. Verfahren nach Anspruch 9, worin das produzierte Hybridplasmid das Plasmid pML3 ist (welches im E. coli-Stamm JM103 (pML3) vorliegt, der bei der American Type Culture Collection, Rockville, Maryland hinterlegt ist unter der Bezeichnung ATCC Nr. 53377).

**Patentansprüche für folgende Vertragsstaaten : AT, ES**

1. Verfahren zur Konstruktion eines Hybridplasmids zur Expression einer DNA-Sequenz, dadurch gekennzeichnet, daß man ein Plasmid, welches eine Hybrid-trp/lac (tac)-Promotor- und Operatorregion einschließlich Transcriptions- und Translationskontrollsignalen umfaßt, wobei in proximaler Position stromabwärts von dieser Region eine einzige BamHI-Schnittstelle angeordnet ist, und das Plasmid außerdem einen selektierbaren, phänotypischen Marker und ein funktionales Replikon für E. coli umfaßt, behandelt, um in die BamHI-Schnittstelle eine DNA-Sequenz zu insertieren, welche von einer 5'-geschnittenen Sau 3A-Schnittstelle und einer 3'-geschnittenen BamHI-Schnittstelle begrenzt ist und eine einzelne EcoRI-Schnittstelle enthält, so daß ein Hybridplasmid mit der Bezeichnung pML3 (welches in dem E. coli-Stamm JM103 (pML3) vorliegt, der bei der American Type Culture Collection, Rockville, Maryland unter der Bezeichnung ATCC Nr. 53377 hinterlegt ist) bereitgestellt wird und eine einzige EcoRI-Schnittstelle in proximaler Position stromabwärts von dem Promotor und eine einzige BamHI-Schnittstelle stromabwärts von der EcoRI-Schnittstelle aufweist, wobei die Promoter- und Operatorregion zur Expression der zwischen den EcoRI- und BamHI-Schnittstellen insertierten DNA befähigt ist.

2. Verfahren nach Anspruch 1, wobei man
(a) ein Plasmid mit der Restriktionsendonuklease EcoRI linearisiert, um kebrige Enden zu produzieren, wobei das Plasmid eine einzige EcoRI-Schnittstelle aufweist, die stromaufwärts von der Hybrid-trp/lac (tac)-Promotor- und Operatorregion liegt und Transcriptions- und Translations-Kontrollsignale enthält, eine einzige BamHI-Schnittstelle in proximaler Position stromabwärts von dieser Region aufweist, wobei diese Region in die BamHI-Schnittstelle insertierte DNA exprimieren kann, und wobei das Plasmid außerdem einen selektierbaren, phänotypischen Marker und ein funktionales Replikon für E. coli umfaßt;
(b) die klebrigen Enden glättet, um ein linearisiertes Plasmid mit bündigen Enden zu erzeugen;
(c) das linearisierte Plasmid gemäß Schritt (b) an den glatten Enden ligiert, um ein zirkularisiertes Plasmid ohne EcoRI-Schnittstelle zu erzeugen; und
(d) in die BamHI-Schnittstelle des zirkularisierten Plasmids gemäß Schritt (c) ein DNA-Segment insertiert, welches durch eine 5'-geschnittene Sau3A-Schnittstelle und eine 3'-geschnittene BamHI-Schnittstelle begrenzt ist und eine einzige EcoRI-Schnittstelle aufweist.

3. Verfahren nach Anspruch 1 oder Anspruch 2, worin das Ausgangsplasmid und das produzierte Hybridplasmid außerdem stromabwärts von der BamHI-Schnittstelle eine Nukleotidsequenz umfaßt, welche für GalK kodiert, wobei die GalK-Sequenz unter Kontrolle der Promotor- und Operatorregion steht.

4. Verfahren nach Anspruch 3, worin der selektierbare, phänotypische Marker des Ausgangsplasmids und des produzierten Hybridplasmids eine für amp$^r$ kodierende, exprimierbare Nukleotidsequenz ist, wobei die amp$^r$-Sequenz stromaufwärts von dem Promotor liegt und im Vergleich zur GalK-Sequenz eine umgekehrte Transcriptionsrichtung aufweist.

5. Verfahren nach einem der Ansprüche 1 bis 4, worin die EcoRI-Schnittstelle des produzierten Hybridplasmids etwa 36 Basenpaare stromabwärts von dem Promotor liegt.

6. Verfahren nach einem der Ansprüche 1 bis 5, worin das Ausgangsplasmid und das produzierte Hybridplasmid Multicopyplasmide sind.

7. Mikroorganismus, transformiert mit dem Plasmid pML3 mit der Bezeichnung E. coli Stamm JM103 (pML3), hinterlegt bei der American Type Culture Collection, Rockville, Maryland, unter der Bezeichnung ATCC Nr. 53377.

8. Verfahren zur Expression einer DNA-Sequenz, wobei man die DNA-Sequenz zwischen der EcoRI-Schnittstelle und der BamHI-Schnittstelle des Plasmids, erhältlich gemäß einem Verfahren der Ansprüche 1 bis 6, insertiert, einen Mikroorganismus mit dem Plasmid transformiert und den Mikroorganismus kultiviert, so daß die DNA-Sequenz exprimiert wird.

9. Verfahren zur Expression eines GRF-Gens, wobei man einen Mikroorganismus kultiviert, der mit dem als pML3 bezeichneten Plasmid (das im E. coli-Stamm JM103 (pML3) bei der American Type Culture Collection, Rockville, Maryland, unter der Bezeichnung ATCC Nr. 53377 hinterlegt ist) transformiert ist, so daß das darin vorliegende GRF-Gen exprimiert wird.

**Revendications**
**Revendications pour les Etats contractants suivants : BE, CH, DE, FR, GB, GR, IT, LI, LU, NL, SE**

1. Plasmide appelé pML3 (tel que présent dans la souche de E. coli JM103 (pML3) déposée auprès de l'American Type Culture Collection, Rockville, Maryland, E.U.A. sous la désignation ATCC n° 53377).

2. Microorganisme tranformé par le plasmide tel que défini dans la revendication 1.

3. Procédé d'expression d'une séquence d'ADN comprenant l'insertion de cette séquence d'ADN entre le site EcoRI et le site BamHI d'un plasmide tel que défini dans la revendication 1, la transformation d'un microorganisme à l'aide du plasmide précité et la culture du microorganisme de manière à exprimer ladite séquence d'ADN.

4. Procédé d'expression d'un gène de GRF comprenant la culture d'un microorganisme transformé à l'aide du plasmide tel que défini dans la revendication 1, de manière à exprimer le gène de GRF qui y est présent.

5. Procédé de construction d'un plasmide hybride, tel que défini dans la revendication 1, caractérisé en ce que l'on traite un plasmide comprenant une région d'opérateur et de promoteur trp/lac, y compris des signaux de commande de la transcription et de la traduction, région en aval proximal de laquelle se situe un site de restriction BamHI, le plasmide comprenant encore un marqueur phénotypique sélectible et un réplicon fonctionnel pour E. coli, de façon à insérer dans le site BamHI une séquence d'ADN liée par un site de restriction Sau 3A 5' coupé et un site de restriction BamHI 3' coupé et incluant un site de restriction EcoRI unique.

6. Procédé suivant la revendication 5, caractérisé en ce que :
   (a) on linéarise un plasmide avec une endonucléase de restriction EcoRI pour engendrer des extrémités collantes, le plasmide possédant un site de restriction EcoRi unique situé en amont d'une région d'opérateur et de promoteur trp/lac (tac) hybride, y compris des signaux de commande de la transcription et de la traduction, un site de restriction BamHI unique en aval proximal de ladite région, cette région étant capable d'exprimer l'ADN introduit dans le site BamHI, le plasmide comprenant encore un marqueur phénotypique sélectible et un réplicon fonctionnel pour E. coli,
   (b) on émousse les extrémités collantes pour obtenir un plasmide linéarisé avec des extrémités émoussées,
   (c) on lie par l'extrémité émoussée le plasmide linéarisé de l'étape (b), de façon à obtenir un plasmide circularisé sans site EcoRI et
   (d) on introduit dans le site BamHI du plasmide circularisé de l'étape (c) un segment d'ADN lié par un site de restriction Sau3A 5' coupé et un site de restriction BamHi 3' coupé et incluant un site de restriction EcoRI unique.

7. Procédé suivant la revendication 5 ou la revendication 6, caractérisé en ce que le plasmide de départ et le plasmide hybride final comprennent encore une séquence de nucléotides expressible codant pour GalK en aval du site BamHI précité, la séquence GalK étant sous la commande de ladite région d'opérateur et de promoteur.

8. Procédé suivant la revendication 6, caractérisé en ce que le marqueur phénotypique sélectible du plasmide de départ et du plasmide hybride final est une séquence de nucléotides expressible codant pour amp$^r$, cette séquence d'amp$^r$ étant en amont du promoteur précité et possédant une direction de transcription opposée à partir de la séquence GalK.

9. Procédé suivant l'une quelconque des revendications 5 à 8, caractérisé en ce que le site EcoRI du plasmide hybride £inal est à environ 36 paires de bases en aval du promoteur précité.

10. Procédé suivant la revendication 9, caractérisé en ce que le plasmide hybride final est pML3 (tel que présent dans la souche de E. coli JM103 (pML3) déposée auprès de l'American Type Culture Collection, Rockville, Maryland, E.U.A. sous la désignation ATCC n° 53377).

**Revendications pour les Etats contractants suivants : AT, ES**

1. Procédé de construction d'un plasmide hybride pour l'expression d'une séquence d'ADN, caractérisé en ce que l'on traite un plasmide comprenant une région d'opérateur et de promoteur trp/lac, y compris des signaux de commande de la transcription et de la traduction, région en aval proximal de laquelle se situe un site de restriction BamHi unique, le plasmide comprenant encore un marqueur phénotypique sélectible et un réplicon fonctionnel pour E. coli, de façon à insérer dans le site BamHI une séquence d'ADN liée par un site de rectriction Sau3A 5' coupé et un site de restriction BamHI 3' coupé et incluant un site EcoRI unique, de manière à créer un plasmide hybride appelé pML3 (tel que présent dans la souche de E. coli JM103 (pML3) déposée auprès de l'American Type Culture Collection, Rockville, Maryland, E.U.A. sous la désignation ATCC n° 53377) et possédant un site de restriction EcoRI unique en aval proximal du promoteur précité et un site BamHI unique en aval du site EcoRI précité, la région d'opérateur et de promoteur étant capable d'exprimer l'ADN introduit entre les sites EcoRI et BamHI.

2. Procédé suivant la revendication 1, caractérisé en ce que :
(a) on linéarise un plasmide avec une endonucléase de restriction EcoRI pour engendrer des extrémités collantes, le plasmide possédant un site de restriction EcoRi unique situé en amont d'une région d'opérateur et de promoteur trp/lac (tac) hybride, y compris des signaux de commande de la transcription et de la traduction, un site de restriction BamHI unique en aval proximal de ladite région, cette région étant capable d'exprimer l'ADN introduit dans le site BamHI, le plasmide comprenant encore un marqueur phénotypique sélectible et un réplicon fonctionnel pour E. coli,
(b) on émousse les extrémités collantes pour obtenir un plasmide linéarisé avec des extrémités émoussées,
(c) on lie par l'extrémité émoussée le plasmide linéarisé de l'étape (b), de façon à obtenir un plasmide circularisé sans site EcoRI et
(d) on introduit dans le site BamHI du plasmide circularisé de l'étape (c) un segment d'ADN lié par un site de restriction Sau3A 5' coupé et un site de restriction BamHI 3' coupé et incluant un site de restriction EcoRI unique.

3. Procédé suivant la revendication 1 ou la revendication 2, caractérisé en ce que le plasmide de départ et le plasmide hybride final comprennent encore une séquence de nucléotides expressible codant pour GalK en aval du site BamHI précité, la séquence GalK étant sous la commande de ladite région d'opérateur et de promoteur.

4. Procédé suivant la revendication 3, caractérisé en ce que le marqueur phénotypique sélectible du plasmide de départ et du plasmide hybride final est une séquence de nucléotides expressible codant pour amp$^r$, cette séquence amp$^r$ étant en amont du promoteur précité et possédant une direction de transcription opposée à partir de la séquence GalK.

5. Procédé suivant l'une quelconque des revendications 1 à 4, caractérisé en ce que le site EcoRI du plasmide hybride final est à environ 36 paires de bases en aval du promoteur précité.

6. Procédé suivant l'une quelconque des revendications 1 à 5, caractérisé en ce que le plasmide de départ et le plasmide hybride final sont des plasmides de multicopie.

7. Microorganisme transformé par le plasmide pML3 et portant la désignation E. coli souche JM103 (pML3) déposée auprès de l'Aremican Type Culture Collection, Rockville, Maryland, E.U.A sous la désignation ATCC n° 53377.

8. Procédé d'expression d'une séquence d'ADN comprenant l'insertion de ladite séquence d'ADN entre le site EcoRI et le site BamHI d'un plasmide que l'on peut obtenir par mise en oeuvre d'un procédé suivant l'une quelconque des revendications 1 à 6, la transformation d'un microorganisme avac le plasmide précité et la culture du microorganisme de manière à exprimer la séquence d'ADN concernée.

9. Procédé d'expression d'un gène de GRF comprenant la culture d'un microorganisme transformé avec le plasmide appelé pML3 (tel que présent dans la souche de E. coli JM103 (pML3) déposée auprès de l'American Type Culture Collection, Rockville, Maryland, E.U.A. sous la désignation ATCC n° 53377) de manière à exprimer le gène de GRF qui y est présent.

FIG. 1

FIG.2

12

*Bam* HI
↓
GGATCC
CCTAGG
↑
*Bam* HI

*Eco* RI - *Sau* 3A
CONVERTER
AATTCGATCG

*Sau* 3A
↑
GATC
CTAG
↓
*Sau* 3A

*Eco* RI
AATTC
      G _____ GRF
      LIGATE
↓
AATTCGATCGAATTC _____
GCTAGCTTAAG _____ GRF
      CUT WITH *Sau* 3A
↓
*pDR 540 Bam* HI
_____ G
      CCTAG
GATCGAATTC _____ GRF
      CTTAAG
      LIGATE
↓
*pDR 540*
      *Eco* RI
_____ GGATCGAATTC _____ GRF
      CCTAGCTTAAG

*Bam* HI SITE DELETED

# FIG. 3